# EUROPÄISCHE PATENTSCHRIFT

(11) **EP 4 101 397 B1**
(45) Veröffentlichungstag und Bekanntmachung des Hinweises auf die Patenterteilung: **04.06.2025**
(21) Anmeldenummer: 22187317.7
(22) Anmeldetag: 09.01.2020
(51) Int. Cl.: A61B 17/12

(54) **OKKLUDEREINFÜHRSYSTEM UND EINFÜHREINHEIT**
OCCLUDER INSERTION SYSTEM AND INSERTION UNIT
SYSTÈME D'INSERTION D'UN DISPOSITIF D'OCCLUSION ET UNITÉ D'INSERTION

(30) Priorität: 10.01.2019 DE 102019100531
(43) Veröffentlichungstag der Anmeldung: 14.12.2022
(62) Teilanmeldung aus: 20700468.0
(73) Patentinhaber: Qatna Medical GmbH, 72379 Hechingen (DE)
(72) Erfinder: Centola, Marcos, 72379 Hechingen-Stein (DE)
(74) Vertreter: DREISS Patentanwälte PartG mbB

(56) Entgegenhaltungen:
- EP-A2- 1 523 957
- WO-A1-2017/157316
- WO-A2-2006/036837
- WO-A2-2007/098232
- DE-A1- 102009 020 012
- US-A1- 2007 135 826
- US-A1- 2007 167 981
- US-A1- 2009 088 795
- US-A1- 2010 114 128
- US-A1- 2012 185 031
- US-A1- 2014 121 755

## Beschreibung

Die Erfindung betrifft ein System zum Einführen eines selbstexpandierbaren Okkluders in einen Patienten und zum Freisetzen des Okkluders in der auricula cordis sinistra des Patienten.

Die auriculae atrii oder Vorhofohren sind Ausstülpungen der Vorhöfe des Herzens bei Säugetieren. Das linke Vorhofohr, medizinisch als auricula cordis sinistra (englisch: Left Atrial Appendage (LAA)) bezeichnet, liegt neben dem Strang der Lungenarterie und ist, insbesondere bei Patienten mit Vorhofflimmern, ein häufiger Entstehungsort für Blutgerinnsel, die zu einem Schlaganfall führen können. Die Verhinderung von Thromben in der auricula cordis sinistra stellt daher eine wirksame Schlaganfallprophylaxe bei gefährdeten Patienten dar.

Für diese Schlaganfallprophylaxe wurden Implantate entwickelt, die in die Ausstülpungen eingesetzt werden und den Zugang beispielsweise über eine Teflonfolie verschließen. In der angelsächsischen Literatur werden diese Implantate als LAA(LAA: Left Atrial Appendage)-Okkluder bezeichnet. Diese Implantate werden in die Ausstülpungen eingesetzt und dort insbesondere mittels Verankerungselementen verankert, so dass sie insbesondere über ihren proximalen Endbereich den Zugang in die Ausstülpungen fluiddicht abschließen. Die Einbringung erfolgt meist über endovaskuläre Techniken, d.h. insbesondere mit einem Einführkatheter, durch den das Implantat an den Einsatzort verbracht wird. Dabei werden die Okkluder insbesondere in volumenreduzierter Form an den Einsatzort verbracht und dort expandiert. Für die Okkluder werden dabei in der Regel selbstexpandierende Materialien verwendet, beispielsweise Formgedächtnislegierungen. Ein derartiger Okkluder ist aus der WO 2015/079023 A1 vorbekannt.

Zum Einführen eines Okkluders in die auricula cordis sinistra eines Patienten, ist es bekannt, Führungsdraht zu verwenden. Dabei wird der Führungsdraht, wie beispielsweise in der WO 2015/079023 A1 offenbart, mittels eines Außengewindes an einem Innengewinde des Okkluders festgeschraubt. Sodann wird der Okkluder an seine Bestimmungsposition in den Bereich der auricula cordis sinistra gebracht. Sobald der Okkluder seine selbstexpandierte Form am Bestimmungsort eingenommen hat, kann der Führungsdraht vom Okkluder gelöst werden, indem der Führungsdraht vom Okkluder abgeschraubt wird. Dabei können insbesondere unerwünschte Drehmomente auf den Okkluder einwirken und im schlimmsten Fall sogar zu einem Ablösen des Okkluders von seiner Bestimmungsposition führen. Folglich besteht ein Bedürfnis, diesen Nachteilen abzuhelfen.

Weiterhin besteht ein Bedürfnis, einen Okkluder möglichst einfach in eine Einführlage, die insbesondere eine komprimierte Lage sein kann, verlagern zu können, um in dieser Einführlage den Okkluder an die auricula cordis sinistra heranzuführen.

Ferner ist aus der DE 10 2009 020 012 A1 und US 5 201 757 A bekannt, dass ein Stent durch eine entgegengesetzte Bewegung zweier Elemente eines Katheters in seine selbstexpandierte Form gebracht werden kann.

In der EP 2 266 465 A1 ist ein Okklusionsinstrument offenbart, wobei beim Einführen keine Anteile über das proximale Ende des Okklusionsinstruments hinausragen.

Aus der US 2017/0056222 A1 ist eine Handhabungseinheit zum Einsetzen einer Prothese bekannt, wobei zwei Elemente der Handhabungseinheit drehbar zueinander verlagerbar sind, sodass sich ein Außenschlauch und ein Innenschlauch relativ zu einander translatorisch bewegen und aus der sich bildenden Lücke die Form expandiert.

Aus der WO 2006/036837 A2 ist ein Einführsystem für einen zweiteiligen Okkluder mit einem Außenschlauch und einem innen geführten Draht bekannt, wobei der Außenschlauch an seinem distalen Ende mittels eines Innengewindes mit dem proximalen Ende des Okkluders zusammenwirkt und der innen geführte Draht an seinem distalen Ende mit dem distalen Ende des Okkluders zusammenwirkt.

In der WO 2017/157316 A1 ist ein Einführsystem für einen faltbaren Okkluder mit einem Außenschlauch und einem Innenschlauch gezeigt, wobei der Außenschlauch mittels eines Innengewindes mit dem proximalen Ende des Okkluders und der Innenschlauch mittels eines Außengewindes mit dem distalen Ende des Okkluders zusammenwirkt.

Die Offenlegungsschrift WO 2006/036837 A2 offenbart ein System zum Einführen eines Okkluders, der zum Verschließen eines offenen Foramen ovale ausgebildet ist. Ein Innenschlauch einer Einführeinheit des Systems ist mit einem distalen Ende des Okkluders bewegungsgekoppelt.

Die Offenlegungsschrift US 2007/0135826 A1 offenbart ein System zum Einführen eines Okkluders, der zum Verschließen der auricula cordis sinistra eines Patienten ausgebildet ist. Ein distales Ende eines Außenschlauchs einer Einführeinheit des Systems ist beim Einführen des Okkluders in dem Okkluder angeordnet.

Der vorliegenden Erfindung liegt folglich insgesamt die Aufgabe zugrunde, den bekannten Stand der Technik weiterzubilden.

Diese Aufgabe wird durch ein System mit den Merkmalen des Anspruchs 1 gelöst.

Vorgeschlagen wird demnach zunächst ein System zum Einführen eines selbstexpandierbaren Okkluders in einen Patienten und zum Freisetzen des Okkluders in der auricula cordis sinistra des Patienten. Das System umfasst den Okkluder und eine Einführeinheit mit einer Antriebseinheit und mit einem Einführkatheter, welcher einen Außenschlauch und einen sich durch den Außenschlauch erstreckenden Innenschlauch umfasst. Ein proximaler Endbereich des Okkluders ist mit dem Außenschlauch bewegungsgekoppelt, während ein distaler Endbereich des Okkluders mit dem Innenschlauch bewegungsgekoppelt ist, wobei ein distales Ende des Außenschlauchs innerhalb des Okkluders angeordnet ist. Die Antriebseinheit wirkt derart mit dem Innenschlauch und dem Außenschlauch des Okkluders zusammen, dass bei deren Betätigung zum einen der Innenschlauch in distaler bzw. proximaler Richtung bewegbar ist und zum anderen der Außenschlauch in proximaler bzw. distaler Richtung bewegbar ist, sodass das proximale Ende und das distale Ende des Okkluders voneinander weg bzw. aufeinander zu bewegbar sind.

Durch die Bewegungskopplung des proximalen Endbereichs des Okkluders mit dem Außenschlauch und des distalen Endbereichs des Okkluders mit dem Innenschlauch, können das proximale Ende und das distale Ende des Okkluders voneinander weg bzw. aufeinander zu bewegt werden. Zum Einführen des Okkluders in einen Patienten und zum Hinführen des Okkluders an die auricula cordis sinistra kann folglich zunächst der Innenschlauch in distaler Richtung bewegt werden, während der Außenschlauch in proximaler Richtung bewegt wird. Dadurch können das proximale Ende und das distale Ende des Okkluders voneinander weg bewegt werden, um so den Okkluder in eine Einführlage zu bringen. In dieser Einführlage ist der Okkluder demnach komprimiert angeordnet, so dass dieser eine vergrößerte Länge aber einen verringerten Durchmesser aufweist. Dadurch kann dieser durch insbesondere Blutgefäße hindurch an die auricula cordis sinistra herangeführt werden.

Zum Freisetzen des Okkluders in der auricula cordis sinistra kann demgegenüber der Innenschlauch in proximaler Richtung bewegt werden, während der Außenschlauch in distaler Richtung bewegt werden kann. Dadurch bewegen sich das proximale Ende und das distale Ende des Okkluders aufeinander zu, wobei der Okkluder insbesondere in seine selbstexpandierte Form strebt. Dabei verringert sich die Länge des Okkluders, während sich sein Durchmesser vergrößert. Im Zuge dieses Freisetzungsvorgangs kommt der Okkluder an der auricula cordis sinistra zur Anlage, um so die auricula cordis sinistra mittels des Okkluders zu verschließen.

Insgesamt kann auf besonders einfache Art und Weise eine Lageänderung des Okkluders herbeigeführt werden, um so den Okkluder zum einen in eine Einführlage zu überführen, in der der Okkluder besonders einfach an die auricula cordis sinistra eines Patienten herangeführt werden kann, und zum anderen den Okkluder freisetzen zu können, wobei dieser insbesondere in seine selbstexpandierte Form strebt und nach der Freisetzung die auricula cordis sinistra dicht verschließt.

Eine vorteilhafte Weiterbildung der Erfindung sieht vor, dass der Außenschlauch in distaler Richtung vor dem Innenschlauch endet. Dadurch kann in besonders einfacher Weise eine Bewegungskopplung des proximalen Endbereichs des Okkluders mit dem Außenschlauch und des distalen Endbereichs des Okkluders mit dem Innenschlauch bereitgestellt werden.

Eine weitere vorteilhafte Ausgestaltung der Erfindung sieht vor, dass der Okkluder einen Rahmen mit einem rohrförmigen proximalen Endbereich umfasst, durch den der Einführkatheter in den Okkluder eingeführt ist. Auf diese Weise kann der Einführkatheter in besonders einfacher Weise in den Okkluder eingeführt werden. Das distale Ende des Außenschlauchs kann dabei in distaler Richtung unmittelbar nach dem rohrförmigen proximalen Endbereich liegen, so dass der Außenschlauch insbesondere mit dem rohrförmigen proximalen Endbereich des Okkluders zur Bewegungskopplung zusammenwirkt. Dabei ist denkbar, dass der Außenschlauch form- bzw. kraftschlüssig mit dem rohrförmigen proximalen Endabschnitt zusammenwirkt. Weiterhin strebt der Okkluder insbesondere in seine selbstexpandierte Form, sodass der Okkluder ausgehend von der Einführlage bei einer Bewegung des Außenschlauchs in distaler Richtung die Bewegung durch das intrinsische Streben in die expandierte Lage unterstützen kann.

Vorteilhaft ist auch, wenn der Okkluder einen topfförmigen distalen Endbereich umfasst, an dem der Innenschlauch des Einführkatheters angeordnet ist. Der distale Endbereich des Innenschlauchs kann folglich insbesondere in den topfförmigen distalen Endbereich des Okkluders eingeführt sein und darin enden. Der topfförmige distale Endbereich umfasst folglich insbesondere einen kreiszylindrischen Mantelabschnitt und einen Bodenabschnitt. Folglich kann insbesondere in distaler Richtung eine Bewegungskoppplung zwischen dem Innenschlauch und dem distalen Endbereich des Okkluders bereitgestellt werden. Dabei ist denkbar, dass der Innenschlauch im distalen topfförmigen Endbereich form- bzw. kraftschlüssig angeordnet ist. Dadurch kann auch dann eine Bewegungskopplung zwischen Innenschlauch und dem distalen topfförmigen Endbereich des Okkluders bereitgestellt werden, wenn der Innenschlauch in proximaler Richtung bewegt wird. Weiterhin strebt der Okkluder insbesondere in seine selbstexpandierte Form, sodass ausgehend von der Einführlage der Okkluder bei einer Bewegung des Innenschlauchs in proximaler Richtung die Bewegung durch das intrinsische Streben in die expandierte Lage unterstützen kann.

Besonders bevorzugt ist, wenn der Okkluder zum Einführen in einen Patienten in eine Einführlage überführbar ist, indem ein distales Ende des Innenschlauchs und ein distales Ende des Außenschlauchs relativ zueinander voneinander weg bewegbar sind, sodass das proximale Ende und das distale Ende des Okkluders voneinander weg bewegbar sind. In dieser Einführlage kann der Okkluder insbesondere einen verringerten Durchmesser aufweisen und damit komprimiert sein. Dabei kann der Okkluder eine vergrößerte Länge aufweisen, so dass in der Einführlage der Okkluder im Vergleich zur Freigabelage zwar einen reduzierten Durchmesser, aber eine größere Länge aufweist. In dieser Einführlage kann der Okkluder besonders einfach zur auricula cordis sinistra herangeführt werden.

Besonders bevorzugt ist dabei, wenn die distalen Enden des Innenschlauchs und des Außenschlauchs zur Freisetzung des Okkluders derart aufeinander zu bewegbar sind, dass das proximale Ende und das distale Ende des Okkluders aufeinander zu bewegbar sind. Dabei ist insbesondere denkbar, dass der Okkluder im Zuge des Freisetzungsvorgangs insbesondere in seine selbstexpandierte Form strebt, sodass ausgehend von der Einführlage der Okkluder bei einer Bewegung des Innenschlauchs in proximaler Richtung bzw. des Außenschlauchs in distaler Richtung der Okkluder intrinsisch in die expandierte Lage strebt.

Dabei kann vorgesehen sein, dass ein mittlerer Teil des Okkluders zwischen dem proximalen Ende und dem distalen Ende seine Lage während der Freisetzung des Okkluders im Wesentlichen unverändert beibehält. Um den Okkluder an seiner Bestimmungsposition im Bereich der auricula cordis sinistra freizusetzen, werden folglich die distalen Enden des Innenschlauchs und des Außenschlauchs aufeinander zu bewegt, so dass sich das proximale Ende und das distale Ende des Okkluders ebenfalls aufeinander zu bewegen. Dadurch dass der Okkluder insbesondere selbstexpandierbar ausgebildet sein kann, kann insbesondere vorgesehen sein, dass im Zuge des Freisetzungsvorgangs der Okkluder in seine selbstexpandierte Lage strebt. In der Endlage kann der Okkluder dabei an einer Innenseite der auricula cordis sinistra zur Anlage kommen und die auricula cordis sinistra dicht verschließen, um so das Risiko einer Thrombenbildung und eines darauf basierenden Schlaganfallrisikos zu senken. In der Endlage kann der Okkluder entweder seine selbstexpandierte Endform einnehmen. Denkbar ist allerdings auch, dass die Endlage des Okkluders, in der dieser zur dichtenden Anlage an die auricula cordis sinistra gelangt, bereits vor Erreichen der selbstexpandierten Endform erreicht ist und sich der Okkluder sodann nicht weiter verformen kann.

Denkbar ist, dass der Okkluder eine Mittelachse aufweist, die sich durch den proximalen Endbereich und den distalen Endbereich hindurch erstreckt. Um diese herum kann der Okkluder verlaufen. Der Okkluder kann dabei in der Einführlage entlang dieser Achse einen Mittelpunkt zwischen dem proximalen Ende und dem distalen Ende aufweisen. Wenn nunmehr der Okkluder freigesetzt wird, kann insbesondere vorgesehen sein, dass das proximale Ende und das distale Ende des Okkluders derart aufeinander zu bewegt werden, dass der Mittelpunkt des Okkluders seine Lage beibehält oder im Wesentlichen beibehält. Dadurch kann eine besonders positionsgenaue Anordnung und Freisetzung des Okkluders an der auricula cordis sinistra bereitgestellt werden. In seiner Endlage kann der Okkluder insbesondere eine kugelförmige Außenkontur aufweisen, so dass es sich bei dem Mittelpunkt auch um den Mittelpunkt der kreisförmigen Außenkontur handeln kann.

Besonders bevorzugt ist weiter, wenn die Antriebseinheit ein erstes Übertragungselement mit einem ersten Übertragungsgewinde, ein zweites Übertragungselement mit einem zweiten Übertragungsgewinde und ein von einer Bedienperson betätigbares Betätigungselement mit einem ersten Antriebsgewinde und einem zweiten Antriebsgewinde aufweist. Dabei ist das erste Übertragungselement mit dem Innenschlauch bewegungsgekoppelt. Das zweite Übertragungselement ist mit dem Außenschlauch bewegungsgekoppelt. Das erste Antriebsgewinde wirkt mit dem ersten Übertragungsgewinde zusammen, während das zweite Antriebsgewinde mit dem zweiten Übertragungsgewinde zusammenwirkt, um die distalen Enden des Außenschlauchs und des Innenschlauchs aufeinander zu bzw. voneinander weg zu bewegen. Auf besonders einfache Art und Weise kann dadurch eine Bewegungskopplung zwischen der Antriebseinheit und dem Außenschlauch bzw. Innenschlauch des Einführkatheters bereitgestellt werden. Dabei können das erste und das zweite Antriebsgewinde einstückig ausgebildet sein. Insbesondere kann das Betätigungselement insgesamt einstückig ausgebildet sein. Bei einer Betätigung des Betätigungselements können folglich zeitgleich die distalen Enden des Außenschlauchs und des Innenschlauchs aufeinander zu bzw. voneinander weg bewegt werden. Dabei kann vorgesehen sein, dass das erste Übertragungsgewinde und das zweite Übertragungsgewinde, sowie das erste und das zweite Antriebsgewinde derart synchron ausgebildet sind, dass bei einer Betätigung des Betätigungselements der Innenschlauch und der Außenschlauch jeweils um dieselbe Distanz in entgegengesetzter Richtung verlagert werden.

Vorteilhafterweise ist das erste Antriebsgewinde als erstes Innengewinde ausgebildet, welches mit dem als Außengewinde ausgebildeten ersten Übertragungsgewinde zusammenwirkt. Ferner ist vorteilhafterweise das zweite Antriebsgewinde als zweites Innengewinde ausgebildet, das mit dem als Außengewinde ausgebildeten zweiten Übertragungsgewinde zusammenwirkt. Das erste und das zweite Antriebsgewinde können einstückig miteinander ausgebildet sein. Insgesamt kann dadurch eine Bewegungskopplung und eine synchrone Bewegung in entgegengesetzte Richtungen auf besonders einfache Art und Weise realisiert werden.

Vorzugsweise weist der Außenschlauch an seinem distalen Endbereich wenigstens einen, insbesondere zwei oder mehr, rastfingerartigen Abschnitt auf, der mit dem proximalen Endbereich des Okkluders zur Bewegungskopplung zusammenwirkt. Auf diese Weise kann eine besonders einfache Bewegungskopplung realisiert werden. Dabei kann der rastfingerartige Abschnitt insbesondere mit dem rohrförmigen proximalen Endanschnitt des Okkluders zusammenwirken und diesen insbesondere zur Bewegungskopplung bei einer Bewegung in proximaler Richtung hintergreifen. Der Außenschlauch kann insbesondere einstückig ausgebildet sein.

Eine besonders bevorzugte Weiterbildung der Erfindung ergibt sich daraus, dass das erste Übertragungselement mit einem am Innenschlauch angeordneten Luer-Anschluss zur Bewegungskopplung mit dem Innenschlauch zusammenwirkt. Am Innenschlauch kann folglich ein Luer-Anschluss angeordnet sein. Dieser kann beispielsweise am ersten Übertragungselement zur Bewegungskopplung angeordnet sein.

Eine besonders bevorzugte Weiterbildung der Erfindung sieht vor, dass der rastfingerartige Abschnitt formschlüssig am proximalen Endbereich des Okkluders angeordnet ist, wenn der Innenschlauch im Okkluder angeordnet ist. Dabei kann insbesondere vorgesehen sein, dass der rastfingerartige Abschnitt elastisch nachgiebig ausgebildet ist und ausgehend von einer Ruhelage nach dem Einführen des Innenschlauchs durch den Außenschlauch hindurch aus der Ruhelage ausgelenkt wird. Wenn der Innenschlauch folglich durch den Außenschlauch hindurchgeführt ist, kann vorgesehen sein, dass der rastfingerartige Abschnitt nicht nach radial innen ausweichen kann und somit ein stabiler Formschluss zwischen dem proximalen Endbereich des Okkluders und dem rastfingerartigen Abschnitt bereitstellbar ist.

Besonders bevorzugt ist dabei, wenn zum Entfernen des Einführkatheters vom Okkluder zunächst der Innenschlauch aus dem Okkluders abziehbar ist. Sobald der Innenschlauch aus dem Okkluder durch den Außenschlauch hindurch abgezogen ist, kann sodann vorgesehen sein, dass der Außenschlauch aus dem Okkluder abziehbar ist.

Folglich kann vorgesehen sein, dass die formschlüssige Verbindung zwischen dem Außenschlauch und dem proximalen Endbereich des Okkluders erst dann aufhebbar ist, wenn der Innenschlauch aus dem Okkluder durch den Außenschlauch hindurch abgezogen ist. Wie oben ausgeführt, können folglich das distale Ende und das proximale Ende des Okkluders zu dessen Freisetzung mittels des Außenschlauchs und des Innenschlauchs aufeinander zu bewegt werden, in dem die distalen Enden des Außenschlauchs und des Innenschlauchs aufeinander zu bewegt werden. Dabei drängt der Okkluder in seine selbstexpandierte Form.

Sobald dieser Vorgang des Aufeinanderzubewegens des proximalen und distalen Endes des Okkluders abgeschlossen ist, kann der Einführkatheter in besonders einfacher Weise vom Okkluder abgezogen werden, indem zunächst der Innenschlauch aus dem Okkluder durch den Außenschlauch hindurch abgezogen wird. Sodann kann der Außenschlauch aus dem Okkluder abgezogen werden, wobei insbesondere der rastfingerartige Abschnitt am distalen Endbereich des Außenschlauchs elastisch in radialer Richtung nach innen verlagert werden kann, um so den Außenschlauch aus dem insbesondere rohrförmigen proximalen Endabschnitt des Okkluders abzuziehen.

Durch dieses Vorgehen kann folglich eine unerwünschte Drehmomentübertragung vom Einführkatheter auf den Okkluder im Wesentlichen vollständig oder insbesondere vollständig unterbunden werden. Dadurch kann das Risiko minimiert bzw. ausgeschlossen werden, dass der Okkluder von seiner Bestimmungsposition an der auricula cordis sinistra gelöst wird, wenn der Einführkatheter vom Okkluder entfernt wird. Nach dem Entfernen des Einführkatheters kann der Okkluder vollends in seine selbstexpandierte Form drängen und dabei insbesondere die auricula cordis sinistra vollständig fluiddicht verschließen.

Vorteilhafterweise weist die Einführeinheit ein Gehäuse auf, das von einer Bedienperson haltbar ist, wobei die Übertragungselemente und das Betätigungselement im bzw. am Gehäuse angeordnet sind. Eine Bedienperson, beispielsweise ein Chirurg, kann folglich das Gehäuse in der Hand halten, so dass das Gehäuse griffartig ausgebildet sein kann. Die Übertragungselemente und/oder das Betätigungselement können im bzw. am Gehäuse angeordnet sein.

Vorzugsweise ist das Betätigungselement drehbar am Gehäuse angeordnet. Dabei kann das Gehäuse eine Mittellängsachse aufweisen und das Betätigungselement kann um die Mittellängsachse drehbar sein. Das Betätigungselement kann dabei insbesondere einstückig ausgebildet sein und insbesondere hohlzylindrisch mit einem Innengewinde ausgebildet sein.

Weitere Einzelheiten und vorteilhafte Ausgestaltungen der Erfindung sind der folgenden Beschreibung zu entnehmen, anhand derer die in den Figuren dargestellte Ausführungsform der Erfindung näher beschrieben und erläutert ist. Es zeigen:
- Figur 1: schematische Draufsicht auf einen an der auricula cordis sinistra angeordneten Okkluder gemäß einer Ausführungsform;
- Figur 2: schematische perspektivische Darstellung des Okkluders gemäß Figur 1;

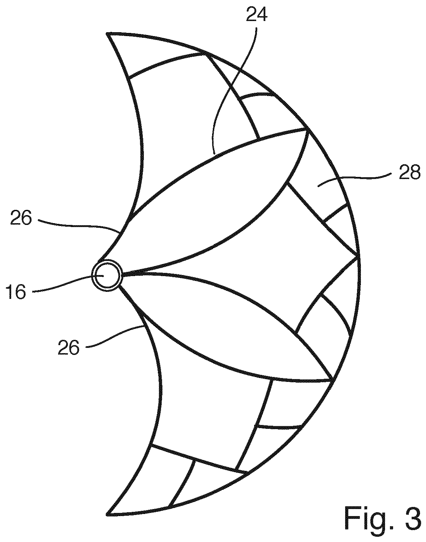

- Figur 3: Draufsicht auf eine Hälfte des Okkluders gemäß Figur 2;
- Figur 4: aufgeschnittene schematische Darstellung eines Rahmenabschnitts des Okkluders gemäß Figur 2;
- Figur 5: perspektivische schematische Darstellung einer Einführeinheit gemäß einer Ausführungsform;
- Figur 6: schematische Querschnittsdarstellung der Einführeinheit gemäß Figur 1 mit daran angeordnetem Okkluder in Einführlage;
- Figur 7: schematischer Querschnitt der Einführeinheit gemäß Figur 1 mit daran angeordnetem Okkluder in einer Konfiguration vor dem Entfernen der Einführeinheit vom Okkluder.
- Figur 8: schematischer Querschnitt eines Bereichs um das proximale Ende des Okkluders herum mit daran angeordneter Einführeinheit;
- Figur 9: Darstellung basierend auf Figur 8, wobei der Innenschlauch der Einführeinheit vom Okkluder entfernt ist; und
- Figur 10: Darstellung eines proximalen Bereichs der Einführeinheit mit von der Einführeinheit gelöstem Luer-Anschluss.

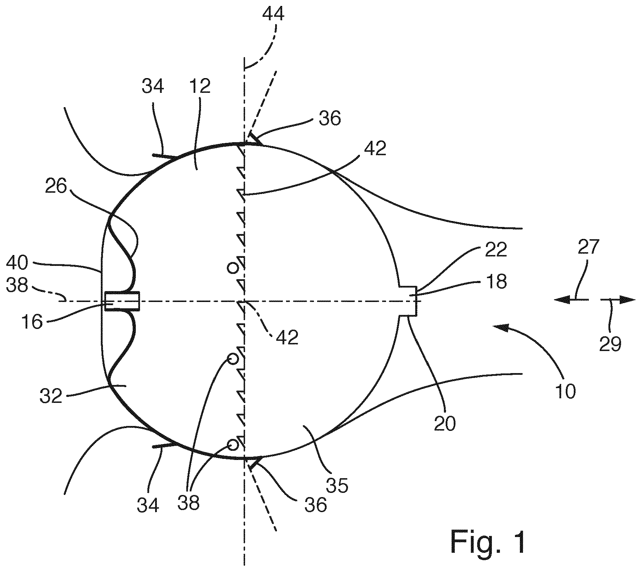

Figur 1 zeigt mit dem Bezugszeichen 10 zunächst schematisch die auricula cordis sinistra eines Patienten. Zur Reduzierung des Schlaganfallrisikos ist dabei ein Okkluder 12 in die auricula cordis sinistra 10 eingesetzt, um den Zugang zur auricula cordis sinistra 10 zu verschließen.

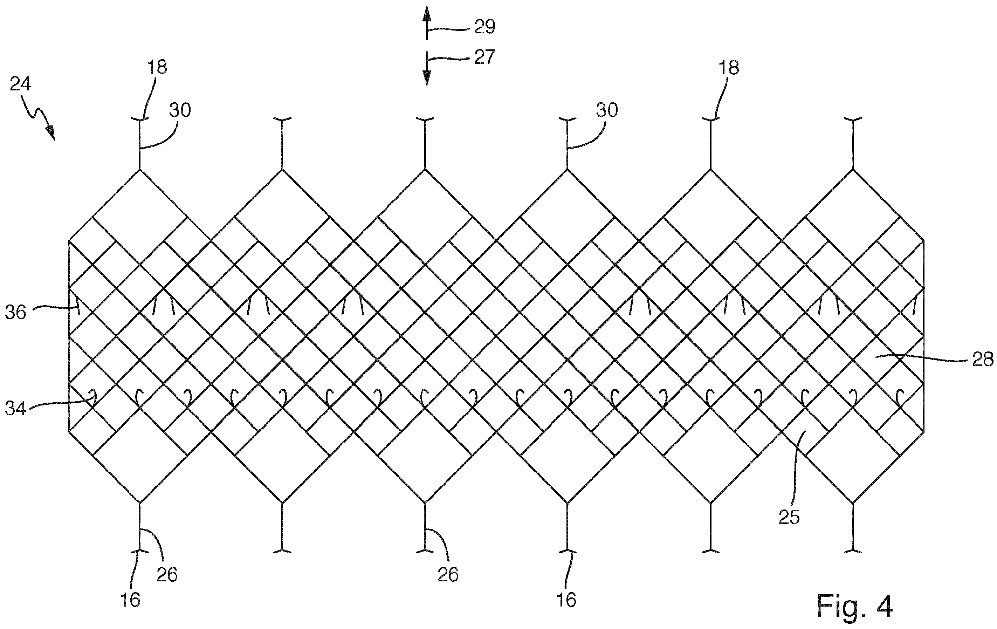

Der Okkluder 12 umfasst zunächst einen einstückig ausgebildeten Rahmen 14. Dieser umfasst einen proximalen rohrförmigen Abschnitt 16 und einen topfförmigen distalen Endabschnitt 18. Der topfförmige distale Endabschnitt 18 umfasst einen kreiszylindrischen Mantelabschnitt 20 und einen Bodenabschnitt 22, um so eine topfförmige Struktur auszubilden. Zwischen den beiden Endabschnitten 16, 18 weist der Okkluder einen netzartigen Rahmenabschnitt 24 auf. Dieser netzartige Rahmenabschnitt 24 ist in Figur 4 in aufgeschnittener Form besonders gut ersichtlich. Ausgehend vom proximalen rohrförmigen Endabschnitt 16 weist der netzartige Rahmenabschnitt 24 zunächst eine Anzahl Stege 26 auf. Diese gehen in ein verzweigendes Netzwerk an Stegen 25 über, um so die Netzstruktur auszubilden. Dabei werden rautenförmige Strukturen 28 ausgebildet. In distaler Richtung 29 laufen die Stege 25 wieder zusammen in einzelne Stege 30, die in den distalen Endabschnitt 18 münden.

Im angeordneten Zustand (vgl. Figur 1) weist der Okkluder 12 eine proximale Hemisphäre 32 und eine distale Hemisphäre 35 auf. Im Bereich der proximalen Hemisphäre 32 sind eine Anzahl erste Verankerungsmittel 34 vorgesehen. Diese erstrecken sich entlang einer Kreislinie entlang des Umfangs und sind hakenförmig ausgebildet mit Endabschnitten, die in proximale Richtung 27 weisen. Im Bereich der distalen Hemisphäre 35 sind zweite Verankerungsmittel 36 ausgebildet. Diese erstrecken sich ebenfalls entlang einer Kreislinie entlang des Umfangs, weisen eine stabartige Form auf und ragen schräg von der Umfangsfläche in proximale Richtung 27 ab. Die Verankerungsmittel 34, 36 sind ebenfalls einstückig mit dem Rahmen 14 ausgebildet.

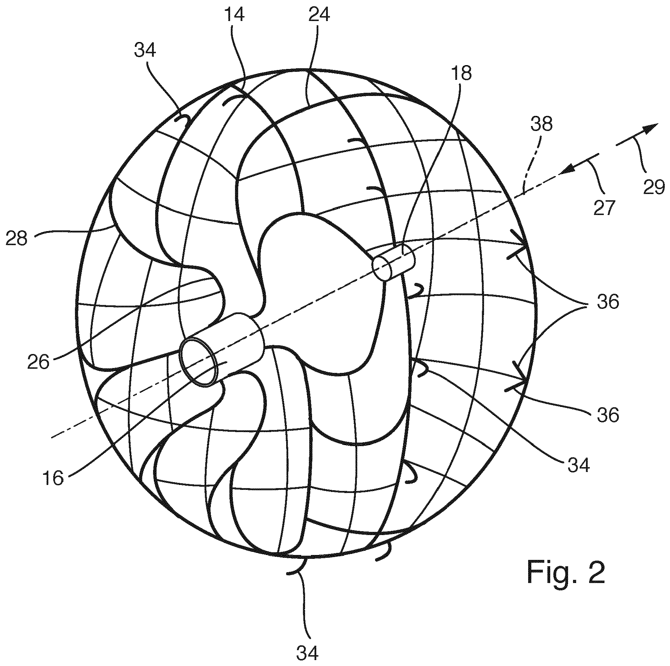

Der Rahmen 14 des Okkluders 12 besteht aus einem selbstexpandierbaren Material, beispielsweise aus einer Formgedächtnislegierung, insbesondere aus einer Nitinol-Legierung. Die dem Okkluder 12 aufgeprägte expandierte Form ist kugelförmig (vgl. Figur 2). Dabei weist der Okkluder 12 eine in proximale bzw. distale Richtung verlaufende Längsachse 38 durch seinen Mittelpunkt auf. Diese Längsachse 38 erstreckt sich auch durch die Mittellängsachse des rohrförmigen proximalen Endabschnitts 16 sowie durch die Mittellängsachse des distalen Endabschnitts 18 (vgl. Figur 2). Die proximale Hemisphäre 32 ist vollständig von einem biologischen Gewebe 40 bedeckt. Dieses biologische Gewebe 40 ist insbesondere als biologische Membran ausgebildet. Insbesondere kann es sich um die Pericardium Membran handeln. Das Gewebe 40 weist Öffnungen auf, sodass die ersten Verankerungsmittel 34 durch die Öffnungen hindurchragen. Ferner weist das Gewebe 40 eine Einführöffnung auf, um einen Einführkatheter durch den proximalen Rohrabschnitt 16 in den Okkluder 12 einzuführen. Wenn der Einführkatheter nach der Freisetzung des Okkluders 12 aus dem Okkluder 12 entfernt worden ist, kann das elastisch nachgiebige Gewebe 40 sich so zusammenziehen, dass die Einführöffnung im Wesentlichen fluiddicht verschlossen wird, so dass insgesamt das Gewebe 40 die proximale Hemisphäre 32 im Wesentlichen fluiddicht abschließt und den Rahmen 14 dabei im Wesentlichen abdeckt. Wie schematisch durch chirurgische Fäden 42 angedeutet, ist das Gewebe mittels PTFE-Fäden am Rahmen 14 angenäht.

In der Nähe der Trennebene 44 der proximalen Hemisphäre 32 und der distalen Hemisphäre 35 sind im Bereich der proximalen Hemisphäre 32 über den Umfang eine Anzahl Röntgenmarker 38 platziert. Diese ermöglichen eine exakte Positionierung des Okkluders 12 in der auricula cordis sinistra. Ein Chirurg kann folglich eine besonders positionsgenaue Platzierung des Okkluders 12 durchführen.

Insgesamt kann durch das Vorsehen des biologischen Gewebes 40 ein Okkluder 12 mit vergleichsweise hoher Biokompatibilität bereitgestellt werden. Dabei kann das biologische Gewebe 40 nach der Anordnung des Okkluders 12 an der auricula cordis sinistra von natürlichem Gewebe des Patienten überwachsen werden. Aufgrund des verwendeten biologischen Gewebes 40 besteht insgesamt eine hohe Biokompatibilität und damit eine erhöhte Erfolgswahrscheinlichkeit, dass ein chirurgischer Eingriff zum Verschließen der auricula cordis sinistra erfolgreich ist.

Im Folgenden wird ein System zum Einführen des Okkluders 12 in einen Patienten und zum Freisetzen des Okkluders 12 in der auricula cordis sinistra 10 des Patienten gemäß einer Ausführungsform dargelegt:

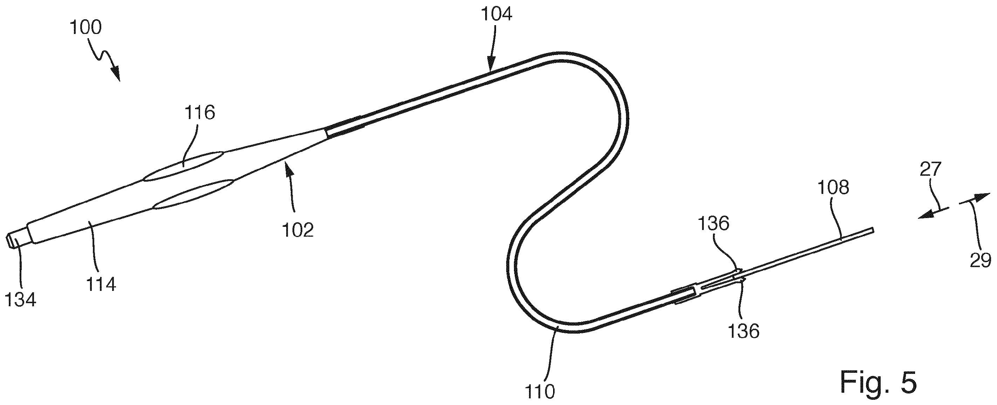

Figur 5 zeigt insgesamt eine Einführeinheit 100. Diese umfasst zum einen eine Antriebseinheit 102 und zum anderen einen Einführkatheter 104. Der Einführkatheter 104 umfasst einen Innenschlauch 108 und einen Außenschlauch 110. Dabei erstreckt sich der Innenschlauch 108 durch den Außenschlauch 110. Ferner endet der Außenschlauch 110 in distaler Richtung 29 vor dem Innenschlauch 108. Die Antriebseinheit 102 umfasst ein Gehäuse 114, das von einer Bedienperson, insbesondere einem Chirurg, in der Hand gehalten werden kann und insgesamt eine längliche Form aufweist.

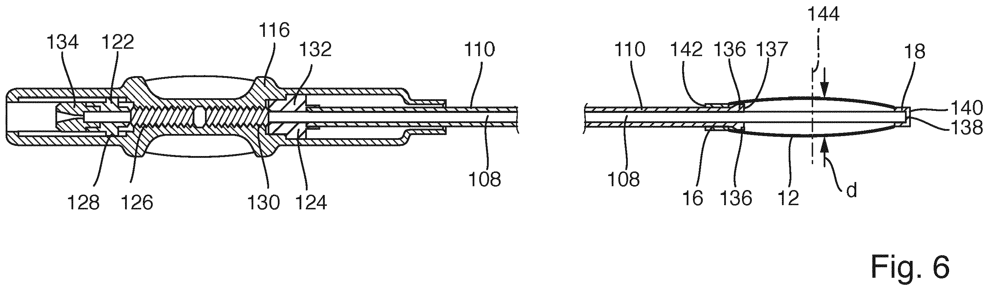

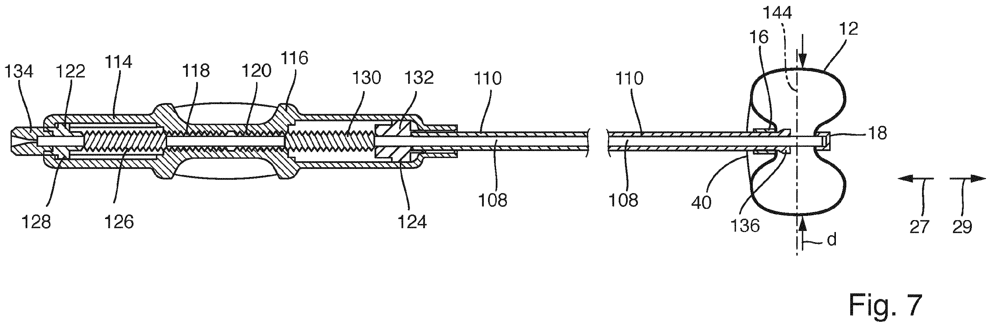

Ferner umfasst die Antriebseinheit 102 ein Betätigungselement 116, das drehbar im Gehäuse 114 angeordnet ist. Dabei ist das Betätigungselement 116 hohl ausgebildet mit einem ersten Antriebsgewinde 118 und einem zweiten Antriebsgewinde 120, wobei das erste Antriebsgewinde 118 proximal zum zweiten Antriebsgewinde 120 ist (vgl. Figuren 6 und 7). Das Betätigungselement 116 ist insbesondere einstückig ausgebildet.

Im Gehäuse 114 sind ferner ein erstes Übertragungselement 122 und ein zweites Übertragungselement 124 angeordnet. Beide Übertragungselemente 122, 124 weisen insgesamt eine schraubenartige Form auf. Dabei weist das erste Übertragungselement 122 einen Gewindeabschnitt 126 und einen Kopfabschnitt 128 auf. Dementsprechend weist das zweite Übertragungselement 124 einen Gewindeabschnitt 130 und einen Kopfabschnitt 132 auf. Beide Übertragungselemente 122, 124 können insbesondere jeweils einstückig ausgebildet sein. Das erste Übertragungselement 122 ist dabei mit dem Innenschlauch 108 bewegungsgekoppelt, während das zweite Übertragungselement 124 mit dem Außenschlauch 110 bewegungsgekoppelt ist. Der Kopfabschnitt 128 des ersten Übertragungselements 122 wirkt dabei mit einem Luer-Anschluss 134 zusammen, der am Innenschlauch 108 befestigt ist. Dabei ist der Luer-Anschluss 134 am Kopfabschnitt 128 des ersten Übertragungselements 122 lösbar angeordnet.

Der Kopfabschnitt 132 des zweiten Übertragungselements 124 wirkt mit dem Außenschlauch 110 zur Bewegungskopplung zusammen. Dabei ist der Außenschlauch 110 lösbar am Kopfabschnitt 132 angeordnet.

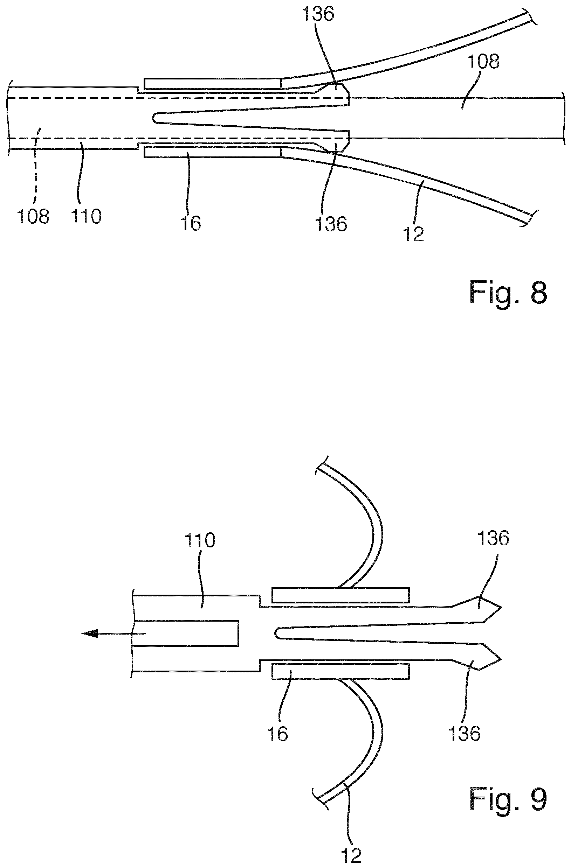

Am distalen Ende des Außenschlauchs 110 weist dieser zwei in Figur 9 besonders deutlich zu erkennende rastfingerartige Endabschnitte 136 auf. Diese sind einstückig mit dem Außenschlauch 110 ausgebildet und elastisch nachgiebig verformbar. Insgesamt ist die Funktionsweise der Einführeinheit 100 sodann wie folgt:
Um den Okkluder 12 in die auricula cordis sinistra 10 einzuführen, wird zunächst der Einführkatheter 104 an dem Okkluder 12 angeordnet, um so ein System aus Einführeinheit 100 und Okkluder 12 auszubilden, um den Okkluder 12 an die auricular cordis sinistra 10 heranzuführen und den Okkluder 12 sodann freizusetzen.

Der Okkluder 12 befindet sich dabei zunächst in seiner selbstexpandierten Form und weist somit eine kugelförmige Außenkontur auf (vgl. Figur 2). Dabei wird der Außenschlauch 110 durch eine nicht gezeigte Einführöffnung im biologischen Gewebe 40 des Okkluders 12 sowie durch den rohrförmigen proximalen Rohrabschnitt 16 in den Okkluder 12 eingeführt. Sodann wird der Innenschlauch 108 durch den Okkluder 12 und den Außenschlauch 110 hindurchgeführt und am distalen topfförmigen Endabschnitt 18 des Okkluders 12 angeordnet. Dabei werden die rastfingerartigen Abschnitte 136 vom Innenschlauch 108 gegen eine elastische Verformung nach radial innen gesichert, so dass die Rastfinger 136 formschlüssig am proximalen rohrförmigen Abschnitt 16 zur Anlage kommen.

Sodann kann von einer Bedienperson, insbesondere von einem Chirurg, das Gehäuse 114 in die Hand genommen werden und das Betätigungselement 116 rotiert werden. Wie in Figur 6 gezeigt, wird dabei das Betätigungselement 116 zunächst so rotiert, dass die kopfartigen Abschnitte 128, 132 der beiden Übertragungselemente 122, 124 aufeinander zu bewegt werden. Dadurch werden das distale Ende 137 des Außenschlauchs 110 sowie das distale Ende 138 des Innenschlauchs 108 voneinander weg bewegt. Dadurch wird das distale Ende 140 des Okkluders 12 in distaler Richtung 29 verlagert, während das proximale Ende 142 des Okkluders in proximaler Richtung 27 verlagert wird. Insgesamt wird dabei der Okkluder 12 in eine Einführlage überführt, so dass der Okkluder 12 insgesamt eine komprimierte Form einnimmt, in der dieser gegenüber der selbstexpandierten Form eine längliche Außenkontur mit einem verringerten Durchmesser d aufweist (vgl. Figur 6). In dieser komprimierten Einführlage kann der Okkluder 12 zusammen mit dem Einführkatheter 104 in ein Blutgefäß eingeführt werden und dann weiter bis zur auricula cordis sinistra an seine Bestimmungsposition herangeführt werden. Die Position des Okkluders 12 kann mittels der Röntgenmarker 38 festgestellt werden. Der Okkluder 12 soll dabei insbesondere im mittleren Bereich, also im Bereich der Röntgenmarker 38, an der auricula cordis sinistra zur Anlage kommen.

Um den Okkluder 12 nunmehr freizusetzen, wird, wie in Figur 7 gezeigt, das Betätigungselement 116 derart rotiert, sodass die kopfartigen Abschnitte 128, 132 voneinander weg bewegt werden.

Dadurch wird der Innenschlauch 108 in proximaler Richtung 27 bewegt, während der Außenschlauch 110 in distaler Richtung 29 bewegt wird. Im Zuge dieser Relativbewegung werden das proximale Ende 142 des Okkluders 12 und das distale Ende 140 des Okkluders 12 aufeinander zu bewegt. Dadurch wird der Durchmesser d des Okkluders 12 größer. Der Okkluder 12 drängt dabei in seine selbstexpandierte Form, sodass das proximale Ende 142 und das distale Ende 140 des Okkluders aufeinander zu drängen.

Die Position des mittleren Teils des Okkluders 12 bleibt dabei insbesondere unverändert, was in den Figuren 6 und 7 anhand der Mittellinie 144 dargestellt ist. Die Mittellinie 144 verläuft dabei durch die Mitte des Okkluders 12 zwischen dem proximalen Ende 142 und dem distalen Ende 140, wenn dieser, wie in Figur 6 gezeigt, an der Bestimmungsposition angelangt ist. Die Mitte des Okkluders 12 bleibt also lagegetreu und damit in seiner Lage unverändert, wenn der Okkluder, wie in Figur 7 dargestellt, freigesetzt wird.

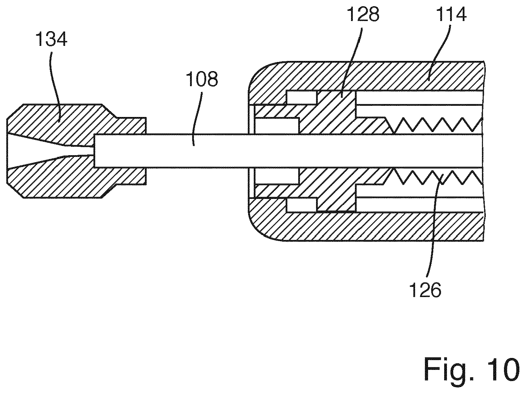

In der in Figur 6 gezeigten Lage ist der Innenschlauch 108 am topfförmigen distalen Endabschnitt 18 des Okkluders 12 kraftschlüssig in proximaler Richtung 27 gehalten. Folglich wird bei einer proximalen Bewegung des Innenschlauchs 108 ebenfalls der distale Endabschnitt 18 des Okkluders 12 in proximaler Richtung 27 bewegt. Wenn der Okkluder 12 dabei in eine Lage, wie in Figur 7 gezeigt, überführt ist, ist dabei die Kraft des Kraftschlusses reduziert, so dass, wie in Figur 10 gezeigt, der Luer-Anschluss 134 vom kopfartigen Abschnitt 128 mit vergleichsweise geringem Kraftaufwand lösbar ist, so dass der Luer-Anschluss 134 und damit der Innenschlauch 108 vom Okkluder 12 durch den Außenschlauch 110 hindurch abziehbar ist.

Daraufhin kann, wie in Figur 10 gezeigt, der Außenschlauch 110 vom Okkluder 12 abgezogen werden, da sich die rastfingerartigen Abschnitte 136 nunmehr elastisch nach radial innen verlagern können. Sodann kann der Okkluder 12 seine Freigabelage einnehmen, in der dieser seine selbstexpandierte Form, wie in Figur 1 gezeigt, einnehmen kann oder jedenfalls in seine selbstexpandierte Endform drängt und damit die auricula cordis sinistra dicht verschließen kann. Wenn der Einführkatheter nach der Freisetzung des Okkluders 12 aus dem Okkluder 12 entfernt worden ist, kann das elastisch nachgiebige Gewebe 40 sich so zusammenziehen, dass die nicht gezeigte Einführöffnung im Gewebe 40 im Wesentlichen fluiddicht verschlossen wird, so dass insgesamt das Gewebe 40 die proximale Hemisphäre 32 im Wesentlichen oder vollständig fluiddicht abschließt und den Rahmen 14 dabei im Wesentlichen oder vollständig abdeckt. Sodann kann Haut des Patienten über das biologische Gewebe 40 wachsen, sodass die auricula cordis sinistra dauerhaft und stabil verschlossen werden kann.

Aufgrund der vorgeschlagenen Konfiguration kann der Einführkatheter 104 besonders einfach vom Okkluder 12 abgezogen werden. Dies insbesondere ohne, oder nahezu ohne, dass ein Drehmoment auf den Okkluder 12 ausgeübt wird. Somit ist die Gefahr reduziert, dass beim Abziehen des Einführkatheters 104 vom Okkluder 12 dieser von seiner Bestimmungsposition in unerwünschter Weise verlagert wird.

## Patentansprüche

1. System zum Einführen eines selbstexpandierbaren Okkluders (12) in einen Patienten und zum Freisetzen des Okkluders (12) in der auricula cordis sinistra (10) des Patienten, das System umfassend den Okkluder (12) und eine Einführeinheit (100) mit einer Antriebseinheit (102) und mit einem Einführkatheter (104), welcher einen Außenschlauch (110) und einen sich durch den Außenschlauch (110) erstreckenden Innenschlauch (108) umfasst, wobei ein proximaler Endbereich (16) des Okkluders (12) mit dem Außenschlauch (110) bewegungsgekoppelt ist, und wobei ein distaler Endbereich (18) des Okkluders (12) mit dem Innenschlauch (108) bewegungsgekoppelt ist, wobei die Antriebseinheit (102) derart mit dem Innenschlauch (108) und dem Außenschlauch (110) zur Freisetzung des Okkluders (12) zusammenwirkt, dass bei deren Betätigung zum einen der Innenschlauch (108) in distaler bzw. proximaler Richtung bewegbar ist und zum anderen der Außenschlauch (110) in proximaler Richtung (27) bzw. distaler Richtung (29) bewegbar ist, sodass das distale Ende (140) und das proximale Ende (142) des Okkluders (12) voneinander weg bzw. aufeinander zu bewegbar sind,
**dadurch gekennzeichnet, dass** beim Einführen des Okkluders (12) ein distales Ende (137) des Außenschlauchs (110) innerhalb des Okkluders (12) angeordnet ist, wobei der Außenschlauch (110) an seinem distalen Endbereich wenigstens einen elastisch nachgiebigen, rastfingerartigen Abschnitt (136) aufweist, wobei dieser nur dann mit dem proximalen Endbereich des Okkluders (12) zusammenwirkt, wenn der Innenschlauch (108) im Außenschlauch (110) angeordnet ist, und wobei der rastfingerartige Abschnitt (136) so ausgebildet ist, dass dieser nach dem Einführen des Innenschlauchs (108) durch den Außenschlauch (110) hindurch aus einer Ruhelage ausgelenkt wird und nicht nach radial innen ausweichen kann.

2. System nach Anspruch 1, wobei der Außenschlauch (110) in distaler Richtung (29) vor dem Innenschlauch (108) endet.

3. System nach einem der vorhergehenden Ansprüche, wobei der Okkluder (12) einen Rahmen (14) mit einem rohrförmigen proximalen Endbereich (16) umfasst, durch den der Einführkatheter (104) in den Okkluder (12) eingeführt ist.

4. System nach einem der vorhergehenden Ansprüche, wobei der Okkluder (12) einen topfförmigen distalen Endbereich (18) umfasst, an dem der Innenschlauch (108) des Einführkatheters (104) angeordnet ist.

5. System nach einem der vorhergehenden Ansprüche, wobei der Okkluder (12) zum Einführen in einen Patienten in eine Einführlage überführbar ist, indem ein distales Ende (138) des Innenschlauchs (108) und das distale Ende (137) des Außenschlauchs (110) relativ zueinander voneinander weg bewegbar sind, sodass das proximale Ende (142) und das distale Ende (140) des Okkluders (12) voneinander weg bewegbar sind.

6. System nach Anspruch 5, wobei die distalen Enden (137, 138) des Innenschlauchs (108) und des Außenschlauchs (110) zur Freisetzung des Okkluders (12) derart aufeinander zu bewegbar sind, dass das proximale Ende (142) und das distale Ende (140) des Okkluders (12) aufeinander zu bewegbar sind, vorzugsweise wobei ein mittlerer Teil (144) des Okkluders (12) zwischen dem proximalen Ende (142) und dem distalen Ende (140) seine Lage während der Freisetzung des Okkluders (12) im Wesentlichen unverändert beibehält.

7. System nach einem der vorhergehenden Ansprüche, wobei die Antriebseinheit (102) ein erstes Übertragungselement (122) mit einem ersten Übertragungsgewinde (126), ein zweites Übertragungselement (124) mit einem zweiten Übertragungsgewinde (130) und ein von einer Bedienperson betätigbares Betätigungselement (116) mit einem ersten Antriebsgewinde (118) und einem zweiten Antriebsgewinde (120) aufweist, wobei das erste Übertragungselement (122) mit dem Innenschlauch (108) bewegungsgekoppelt ist, und wobei das zweite Übertragungselement mit dem Außenschlauch (110) bewegungsgekoppelt ist, und wobei das erste Antriebsgewinde (118) mit dem ersten Übertragungsgewinde (126) zusammenwirkt und das zweite Antriebsgewinde (120) mit dem zweiten Übertragungsgewinde (130) zusammenwirkt, um die distalen Enden (137, 138) des Außenschlauchs (110) und des Innenschlauchs (108) aufeinander zu bzw. voneinander weg zu bewegen.

8. System nach Anspruch 7, wobei das erste Antriebsgewinde (118) als erstes Innengewinde ausgebildet ist, das mit dem als Außengewinde ausgebildeten ersten Übertragungsgewinde (126) zusammenwirkt, und wobei das zweite Antriebsgewinde (120) als zweites Innengewinde ausgebildet ist, das mit dem als Außengewinde ausgebildeten zweiten Übertragungsgewinde (130) zusammenwirkt.

9. System nach einem der Ansprüche 7 und 8, wobei das erste Übertragungselement mit einem am Innenschlauch (108) angeordneten Luer-Anschluss (134) zur Bewegungskopplung mit dem Innenschlauch (108) zusammenwirkt.

10. System nach einem der vorhergehenden Ansprüche, wobei der rastfingerartige Abschnitt (136) formschlüssig mit dem proximalen Endbereich (16) des Okkluders (12) zusammenwirkt, wenn der Innenschlauch (108) im Okkluder (12) angeordnet ist.

11. System nach einem der vorhergehenden Ansprüche, wobei zum Entfernen des Einführkatheters vom Okkluder (12) zunächst der Innenschlauch (108) aus dem Okkluder (12) abziehbar ist, und sodann der Außenschlauch (110) aus dem Okkluder (12) abziehbar ist, sobald der Innenschlauch (108) aus dem Okkluder (12) durch den Außenschlauch (110) hindurch abgezogen ist.

12. System nach einem der vorhergehenden Ansprüche 7 bis 9, wobei die Einführeinheit (102) ein Gehäuse (114) aufweist, das von einer Bedienperson haltbar ist, und wobei die Übertragungselemente und das Betätigungselement im bzw. am Gehäuse (114) angeordnet sind.

13. System nach Anspruch 7, wobei das Betätigungselement (116) drehbar am Gehäuse angeordnet ist.

## Claims

1. System for inserting a self-expandable occluder (12) into a patient and for releasing the occluder (12) in the left atrial appendage (10) of the patient, the system comprising the occluder (12) and an insertion unit (100) which has a drive unit (102) and has an insertion catheter (104) comprising an outer tube (110) and an inner tube (108) extending through the outer tube (110), wherein a proximal end region (16) of the occluder (12) is movement-coupled to the outer tube (110), and wherein a distal end region (18) of the occluder (12) is movement-coupled to the inner tube (108), wherein, in order to release the occluder (12), the drive unit (102) interacts with the inner tube (108) and the outer tube (110) such that, when actuated, the inner tube (108) can be moved in a distal or proximal direction and the outer tube (110) can be moved in the proximal direction (27) or distal direction (29), so that the distal end (140) and the proximal end (142) of the occluder (12) can be moved away from or toward one another, **characterized in that** during insertion of the occluder (12), a distal end (137) of the outer tube (110) is disposed inside the occluder (12), wherein the outer tube (110) comprises at least one elastically yielding latching finger-like portion (136) at its distal end region, wherein the latching finger-like portion (136) cooperates with the proximal end region of the occluder (12) only when the inner tube (108) is disposed inside the outer tube (110), and wherein the latching finger-like portion (136) is configured such that, after the insertion of the inner tube (108) through the outer tube (110), it is deflected from a rest position and cannot deflect radially inward.

2. System according to claim 1, wherein the outer tube (110) ends in front of the inner tube (108) in the distal direction (29).

3. System according to any of the preceding claims, wherein the occluder (12) comprises a frame (14) having a tubular proximal end portion (16) through which the insertion catheter (104) is inserted into the occluder (12).

4. System according to any of the preceding claims, wherein the occluder (12) comprises a pot-shaped distal end region (18) on which the inner tube (108) of the insertion catheter (104) is arranged.

5. System according to any of the preceding claims, wherein, in order to be inserted into a patient, the occluder (12) can be transferred into an insertion position by moving a distal end (138) of the inner tube (108) and the distal end (137) of the outer tube (110) relative to one another and away from one another, so that the proximal end (142) and the distal end (140) of the occluder (12) can be moved away from one another.

6. System according to claim 5, wherein, in order to release the occluder (12), the distal ends (137, 138) of the inner tube (108) and the outer tube (110) can be moved toward one another such that the proximal end (142) and the distal end (140) of the occluder (12) can be moved toward one another, preferably wherein a central part (144) of the occluder (12) between the proximal end (142) and the distal end (140) keeps its position substantially unchanged during the release of the occluder (12).

7. System according to any of the preceding claims, wherein the drive unit (102) has a first transmission element (122) that has a first transmission thread (126), a second transmission element (124) that has a second transmission thread (130), and an actuating element (116) that can be actuated by an operator and has a first drive thread (118) and a second drive thread (120), wherein the first transmission element (122) is movement-coupled to the inner tube (108), and wherein the second transmission element is movement-coupled to the outer tube (110), and wherein the first drive thread (118) interacts with the first transmission thread (126) and the second drive thread (120) interacts with the second transmission thread (130) in order to move the distal ends (137, 138) of the outer tube (110) and the inner tube (108) toward or away from one another.

8. System according to claim 7, wherein the first drive thread (118) is designed as a first internal thread which interacts with the first transmission thread (126) that is designed as an external thread, and wherein the second drive thread (120) is designed as a second internal thread which interacts with the second transmission thread (130) which is designed as an external thread.

9. System according to claim 7 or 8, wherein the first transmission element interacts with a Luer connector (134) arranged on the inner tube (108) for the purpose of movement coupling to the inner tube (108).

10. System according to any of the preceding claims, wherein the latching finger-like portion (136) interacts with the proximal end region (16) of the occluder (12) in a form-fitting manner when the inner tube (108) is arranged in the occluder (12).

11. System according to any of the preceding claims, wherein, in order to remove the insertion catheter from the occluder (12), the inner tube (108) can first be pulled out of the occluder (12), and then the outer tube (110) can be pulled out of the occluder (12) as soon as the inner tube (108) has been pulled out of the occluder (12) through the outer tube (110).

12. System according to any of claims 7 to 9, wherein the insertion unit (102) has a housing (114) which can be held by an operator, and wherein the transmission elements and the actuating element are arranged in or on the housing (114).

13. System according to claim 7, wherein the actuating element (116) is rotatably arranged on the housing.

## Revendications

1. Système pour introduire un dispositif d'occlusion (12) auto-expansible dans un patient et pour libérer le dispositif d'occlusion (12) dans l'auricula cordis sinistra (10) du patient, le système comprenant le dispositif d'occlusion (12) et une unité d'introduction (100) avec une unité d'entraînement (102) et avec un cathéter d'introduction (104), lequel comprend un tuyau extérieur (110) et un tuyau intérieur (108) s'étendant à travers le tuyau extérieur (110), dans lequel une zone d'extrémité proximale (16) du dispositif d'occlusion (12) est couplée en mouvement au tuyau extérieur (110), et dans lequel une zone d'extrémité distale (18) du dispositif d'occlusion (12) est couplée en mouvement au tuyau intérieur (108), dans lequel l'unité d'entraînement (102) coopère avec le tuyau intérieur (108) et le tuyau extérieur (110) pour libérer le dispositif d'occlusion (12) de telle sorte que, lors de son actionnement, d'une part le tuyau intérieur (108) peut être déplacé dans la direction distale ou proximale et d'autre part le tuyau extérieur (110) peut être déplacé dans la direction proximale (27) ou la direction distale (29), de sorte que l'extrémité distale (140) et l'extrémité proximale (142) du dispositif d'occlusion (12) peuvent être éloignées l'une de l'autre ou rapprochées l'une de l'autre, **caractérisé en ce que**, lors de l'introduction du dispositif d'occlusion (12), une extrémité distale (137) du tuyau extérieur (110) est disposée à l'intérieur du dispositif d'occlusion (12), dans lequel le tuyau extérieur (110) présente sur sa zone d'extrémité distale au moins une section du type doigt d'encliquetage (136), élastiquement flexible, dans lequel celle-ci ne coopère avec la zone d'extrémité proximale du dispositif d'occlusion (12) que lorsque le tuyau intérieur (108) est disposé dans le tuyau extérieur (110), et dans lequel la section du type doigt d'encliquetage (136) est réalisée de telle sorte que celle-ci est défléchie à partir d'une position de repos après l'introduction du tuyau intérieur (108) à travers le tuyau extérieur (110) et ne peut pas dévier radialement vers l'intérieur.

2. Système selon la revendication 1, dans lequel le tuyau extérieur (110) se termine dans la direction distale (29) avant le tuyau intérieur (108).

3. Système selon l'une quelconque des revendications précédentes, dans lequel le dispositif d'occlusion (12) comprend un cadre (14) avec une zone d'extrémité proximale tubulaire (16) à travers laquelle le cathéter d'introduction (104) est introduit dans le dispositif d'occlusion (12).

4. Système selon l'une quelconque des revendications précédentes, dans lequel le dispositif d'occlusion (12) comprend une zone d'extrémité distale en forme de pot (18) sur laquelle est disposé le tuyau intérieur (108) du cathéter d'introduction (104).

5. Système selon l'une quelconque des revendications précédentes, dans lequel le dispositif d'occlusion (12) peut être amené dans une position d'introduction pour être introduit dans un patient du fait qu'une extrémité distale (138) du tuyau intérieur (108) et l'extrémité distale (137) du tuyau extérieur (110) peuvent être éloignées l'une de l'autre, de sorte que l'extrémité proximale (142) et l'extrémité distale (140) du dispositif d'occlusion (12) peuvent être éloignées l'une de l'autre.

6. Système selon la revendication 5, dans lequel les extrémités distales (137, 138) du tuyau intérieur (108) et du tuyau extérieur (110) peuvent être rapprochées l'une de l'autre pour libérer le dispositif d'occlusion (12), de telle sorte que l'extrémité proximale (142) et l'extrémité distale (140) du dispositif d'occlusion (12) puissent être rapprochées l'une de l'autre, de préférence dans lequel une partie centrale (144) du dispositif d'occlusion (12) entre l'extrémité proximale (142) et l'extrémité distale (140) conserve sa position sensiblement inchangée pendant la libération du dispositif d'occlusion (12).

7. Système selon l'une quelconque des revendications précédentes, dans lequel l'unité d'entraînement (102) présente un premier élément de transmission (122) avec un premier filetage de transmission (126), un deuxième élément de transmission (124) avec un deuxième filetage de transmission (130) et un élément d'actionnement (116) pouvant être actionné par un opérateur avec un premier filetage d'entraînement (118) et un deuxième filetage d'entraînement (120), dans lequel le premier élément de transmission (122) est couplé en mouvement au tuyau intérieur (108) et dans lequel le deuxième élément de transmission est couplé en mouvement au tuyau extérieur (110), et dans lequel le premier filetage d'entraînement (118) coopère avec le premier filetage de transmission (126) et le deuxième filetage d'entraînement (120) coopère avec le deuxième filetage de transmission (130) pour rapprocher et éloigner l'une de l'autre les extrémités distales (137, 138) du tuyau extérieur (110) et du tuyau intérieur (108).

8. Système selon la revendication 7, dans lequel le premier filetage d'entraînement (118) est réalisé sous la forme d'un premier filetage intérieur qui coopère avec le premier filetage de transmission (126) réalisé sous la forme d'un filetage extérieur, et dans lequel le deuxième filetage d'entraînement (120) est réalisé sous la forme d'un deuxième filetage intérieur qui coopère avec le deuxième filetage de transmission (130) réalisé sous la forme d'un filetage extérieur.

9. Système selon l'une quelconque des revendications 7 et 8, dans lequel le premier élément de transmission coopère avec un raccord Luer (134) disposé sur le tuyau intérieur (108) pour le couplage en mouvement au tuyau intérieur (108).

10. Système selon l'une quelconque des revendications précédentes, dans lequel la section du type doigt d'encliquetage (136) coopère par complémentarité de formes avec la zone d'extrémité proximale (16) du dispositif d'occlusion (12) lorsque le tuyau intérieur (108) est disposé dans le dispositif d'occlusion (12).

11. Système selon l'une quelconque des revendications précédentes, dans lequel, pour retirer le cathéter d'introduction du dispositif d'occlusion (12), le tuyau intérieur (108) peut d'abord être enlevé du dispositif d'occlusion (12), puis le tuyau extérieur (110) peut être enlevé du dispositif d'occlusion (12) dès que le tuyau intérieur (108) est enlevé du dispositif d'occlusion (12) à travers le tuyau extérieur (110).

12. Système selon l'une quelconque des revendications précédentes 7 à 9, dans lequel l'unité d'introduction (102) présente un boîtier (114) qui peut être tenu par un opérateur, et dans lequel les éléments de transmission et l'élément d'actionnement sont disposés dans ou sur le boîtier (114).

13. Système selon la revendication 7, dans lequel l'élément d'actionnement (116) est disposé de manière rotative sur le boîtier.
